# EUROPÄISCHE PATENTANMELDUNG

(11) **EP 1 051 923 A2**
(43) Veröffentlichungstag der Anmeldung: **15.11.2000**
(21) Anmeldenummer: 00109528.0
(22) Anmeldetag: 04.05.2000
(51) Int. Cl.: A41B 9/04, A41F 11/16

(54) **Elastiches Band und Verfahren zu dessen Herstellung**

(30) Priorität: 04.05.1999 DE 19920483
(71) Anmelder: Berger GmbH, D-91757 Treuchtlingen (DE)
(72) Erfinder: Schülein, Fritz, 91757 Treuchtlingen (DE); Hahn, Siegfried, 4050 Traun (AT)
(74) Vertreter: Fischer, Matthias, Dipl.-Ing.

(57) **Zusammenfassung**

Es wird ein elastisches Band (2) mit Antirutschbeschichtung, insbesondere für elastische Strümpfe vorgeschlagen, das dadurch gekennzeichnet ist, daß auf einer Seite des Bandes (2) im wesentlichen quer über die Bandbreite verlaufende, zueinander beabstandete Haftmittelstreifen angeordnet sind. In einer Weiterbildung verlaufen die Haftmittelstreifen (Streifenabschnitte) (6, 6a, 6b, 6c) im wesentlichen quer zur Bandlängsachse und sind unterbrochen. In einer anderen Weiterbildung verlaufen zwei benachbarte Haftmittelstreifen (10, 12) unter entgegengesetzten Winkeln (α, -α) zur Bandlängsachse.

## Beschreibung

Die Erfindung betrifft ein elastisches Band mit Antirutschbeschichtung, insbesondere für Kleidungsstücke, insbesondere für elastische z. B. halterlose Strümpfe.

Es sind elastische Bänder für therapeutische Strümpfe (Gummistrümpfe) bekannt, die direkt auf der Haut getragen werden. Diese Strümpfe haben in der Regel ein Abschlußband in Form eines elastischen Bandes, das auf der zur Hautoberfläche zeigenden Breitseite mit einer Antirutschbeschichtung versehen ist, um ein Herunterrutschen des Strumpfes zu verhindern. Sofern die Beschichtung des Bandes großflächig ausgeführt wird, ergeben sich aufgrund der dadurch verursachten schlechten Belüftung des entsprechenden Hautgebietes beim Träger großflächige Druckstellen und infolge des unbelüfteten Milieus Hautreizungen, die zu Allergien führen können. Diesem Nachteil hat man abgeholfen, indem man statt der großflächigen Beschichtung nur noch punktförmige Beschichtungen verwendet hat. Der sich dadurch jedoch neu ergebende Nachteil besteht darin, daß sich das Band bei der geringsten streifenden Berührung von der Abschlußkante her umschlagen läßt und auch umgeschlagen bleibt. Aufgrund dieses Umschlagen des Bandes ergeben sich Knicke, die nur sehr schlecht aus dem Band selbst wieder herauszubekommen sind. Durch Umschlagen oder Einrollen des Bandes entlang der Bandlängsachse entsteht ein unerwünschter sog. Kordeleffekt, der insbesondere bei Strumpfbändern zu Einschnürungen mit daraus resultierendem Blutstau oder anderen Nachteilen führen kann. Allein aufgrund der Tatsache, daß das Band zu einem großen Teil aus elastischen (Kautschuk, Gummi und dgl.) Fäden besteht, die ein Glattbügeln nicht zulassen, verschleißt das Band nach leichtem Umklappen und wegen der dadurch entstehenden Knickungen schneller.

Der Erfindung liegt nun die Aufgabe zugrunde, ein einerseits rutschfestes elastisches Band zu schaffen, das andererseits jedoch ausreichende Belüftung der Hautkontaktfläche zuläßt und gegen ein Umklappen widerstandsfähiger und damit verschleißfester ist, als aus dem Stand der Technik bekannte Bänder.

Die Aufgabe wird gelöst mit einem elastischen Band gemäß Anspruch 1. Die im wesentlichen quer über die Bandbreite verlaufenden, zueinander beabstandeten Haftmittelstreifen stabilisieren das Band über seine Breite und erschweren das unerwünschte Einrollen oder Knicken des Bandes quer zur Bandlängsachse. Der Tragekomfort wird dadurch gegenüber bekannten Bändern erheblich gesteigert. Die Breite der Haftmittelstreifen kann gegen über bekannten Bändern verringert werden, was die Tragequalität zusätzlich erhöht. in einer vorteilhaften Weiterbildung der Erfindung sind die Haftmittelstreifen (Streifenabschnitte) (6, 6a, 6b, 6c) im wesentlichen quer zur Bandlängsachse verlaufend und unterbrochen angeordnet. Die unterbrochenen Haftmittelstreifen oder Streifenabschnitte haben sich vorteilhafterweise ebenfalls als Rückstellkraft aufweisende Formelemente herausgestellt, die im Vergleich zu herkömmlichen, beispielsweise punktuell beschichteten elastischen Bändern eine wesentlich höhere Rückstellkraft und damit Formhaltigkeit aufweisen. Neben der vorteilhaften Rückstellkraft wird auch die einwandfreie Belüftung des von dem elastischen Band bedeckten Hautbereiches gewährleistet.

In einer vorteilhaften Weiterbildung der Erfindung sind die Streifenabschnitte benachbarter Streifen bezüglich der Bandlängsachse zueinander versetzt angeordnet. Dies ergibt eine im Prinzip bienenwabenähnliche Struktur des Bandes, die die zuvor genannte Rückstellkraft noch einmal wesentlich verbessert.

In einer anderen vorteilhaften Weiterbildung der Erfindung sind jeweils zwei benachbarte Haftmittelstreifen unter entgegengesetzten Winkeln zur Bandlängsachse verlaufend angeordnet. Diese Anordnung ermöglicht zusätzlich zum Hafteffekt (Reib- oder Kraftschluß) auch einen Formschluß. Zwei benachbarte Haftmittelstreifen, die sich, unter entgegengesetzten Winkeln zur Bandlängsachse von einer Bandkante zur anderen Bandkante verlaufend, voneinander "entfernen", wirken wie ein Keil, wenn das Band quer zur Bandlängsachse belastet wird. Zwischen den beiden Haftmittelstreifen befindliche Hautpartien werden dann durch den Keileffekt komprimiert und bilden dadurch eine Erhebung, die wiederum als Hindernis gegen ein Abrutschen oder Knicken des Bandes wirkt.

Die Erfindung betrifft auch ein Kleidungsstück, insbesondere einen Strumpf, insbesondere einen therapeutischen Strumpf, gekennzeichnet durch wenigstens ein Abschlußband nach einem der Ansprüche 1 bis 4.

Diese Erfindung ist insbesondere bei sogenannten halterlosen Strümpfen, die nicht nur als Kniestrümpfe, sondern als 3/4-lang im Oberschenkelbereich endende Strümpfe verwendet werden, von großem Vorteil. Die hier bisher bekannten halterlosen Strümpfe haben zur Bandlängsachse parallele, z. B. etwa 15 mm breite Beschichtungsstreifen, die unschöne Druckstellen und auch Allergiereaktionen, wie auch oben erwähnt, hervorrufen können. Die Ausstattung halterloser Strümpfe mit dem erfindungsgemäßen Band vermeidet derartige Druckstellen und damit einhergehende Nachteile.

Das erfindungsgemäße Band kann auch bei anderen Kleidungsstücken, wie z.B. Radfahranzügen oder Radfahrhosen eingesetzt werden, deren Abschlußkanten im Bereich der Ober-oder Unterschenkel rutschfest mit der Haut in Kontakt stehen sollen, wobei die Hautkontaktflächen aufgrund der bei sportlicher Betätigung besonders hohen Schweißbildung besonders gefährdet sind. Die Erfindung kann also vorteilhaft für alle Arten von Kleidungsstücken zum Einsatz kommen, in denen gleichzeitig Rutschfestigkeit des Textils auf der Haut und gute Durchlüftung der Hautoberfläche gewährleistet sein sollen.

Zum besseren Verständnis der Erfindung wird diese im Anschluß anhand eines auch in der Zeichnung dargestellten Ausführungsbeispiels kurz beschrieben.
- Figur 1: zeigt einen Abschnitt eines erfindungsgemäßen Bandes in etwa in Original-größe.
- Figur 2: zeigt das in Fig. 1 dargestellte Band geschnitten entlang der Linie II-II (ohne Hintergrund).
- Figur 3: zeigt ein elastisches Band, das dadurch gekennzeichnet ist, daß jeweils zwei benachbarte Haftmittelstreifen unter entgegengesetzten Winkeln zur Bandlängsachse verlaufen.

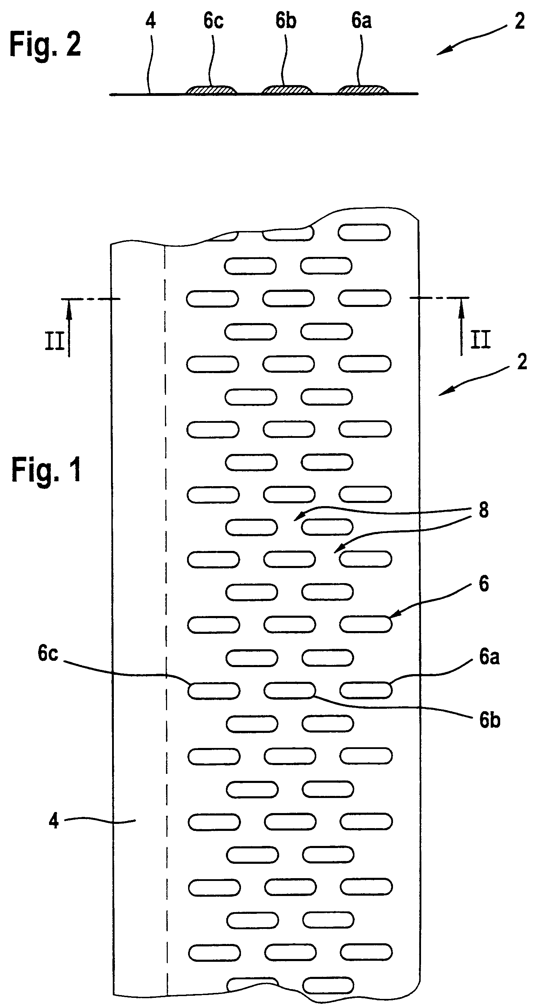

In Fig. 1 wird ein Band 2 gezeigt, dessen Bandlängsachse in der Zeichnung von oben nach unten verläuft. Das Band 2 wird mit seinem (in dieser Darstellung) linken Randstreifen 4 an einem Abschluß eines nicht gezeigten Strumpfes oder Kleidungsstückes angenäht, und zwar in der Orientierung, in der die zum Betrachter zeigende Breitseite des erfindungsgemäßen Bandes 2 mit darauf befindlichen unterbrochenen Haftmittelstreifen 6 auf die innere oder "linke" Seite des Kleidungsstückes weist. Die Haftmittelstreifen 6 sind in Streifenabschnitte, beispielsweise 6a, 6b und 6c, unterteilt. Es ist leicht zu erkennen, daß zwischen den Haftmittelstreifen 6 befindliche Zwischenräume 8 ausreichend Belüftung auf die Hautpartie im Bereich der Kontaktstelle des erfindungsgemäßen elastischen Bandes mit dem Träger des Kleidungsstückes zuläßt. Selbstverständliche Voraussetzung ist hier natürlich, daß das Gewebe des Bandes selbst luftdurchlässig ist. Die Haftmittelstreifen 6 bestehen beispielsweise aus dem Fachmann bekanntem Industriesilikon, das mittels bekannter Vorrichtungen, im vorliegenden Fall jedoch unter Verwendung einer Matrize, die die in Figur 1 gezeigte Kontur der Haftmittelstreifen zuläßt, in etwa bei Raumtemperatur aufgebracht und werden mit bekannten Mitteln zur weiteren Verarbeitung in kurzer Zeit an der Oberfläche in grifftrockenen Zustand gebracht, so daß man ein beschichtetes Band kurz nach der Beschichtung in loser Schüttung lagern kann, ohne daß die Beschichtung zerstört wird. Das Aufbringen des Haftmittels ist vergleichbar mit dem dem Fachmann bekannten Stoffdruckverfahren.

Wie schon im vorderen Teil der Beschreibung angesprochen wurde, ist die Anordnung der Antirutschbeschichtung nach der Erfindung tatsächlich sehr gut geeignet, ein Umschlagen des Bandes zu verhindern. Ganz exakt betrachtet wird nicht das Umschlagen als solches verhinden, sondern das bleibende Umschlagen. Wird nämlich das elastische Band durch eine lokale unbeabsichtigte Berührung "abgestreift", so stellt sich das Band gewissermaßen wieder in seine ursprüngliche Position zurück, sofern der Strumpf beispielsweise nicht aktiv ausgezogen wird.

Die Auswahl der geometrischen Verhältnisse des hier dargestellten Bandes und der Haftmittelstreifen ist nur beispielhaft. Selbstverständlich kann die Erfindung in jeder anderen sinngemäßen geometrischen Form und Zuordnung ausgeführt werden, ohne daß die Vorteile des erfindungsgemäßen Bandes verloren gehen. Beispielsweise ist der Annähstreifen 4, in Figur 1 auf der linken Seite dargestellt und begrenzt durch die linke Bandkante, und die dazu parallel verlaufende gestrichelte Kante nicht unbedingt auf diese Breite oder Form beschränkt. Hier sind sämtliche in der Mode üblichen Applikationen und dgl. denkbar. Dasselbe gilt auch für die rechte und schlußendlich äußere Kante des mit dem elastischen Band bestückten Kleidungsstückes. Die aus Figur 1 erkennbare, etwa bienenwabenförmige Anordnung zweier benachbarter Streifen von Streifenabschnitten stabilisiert und vergrößert die Umschlagwiderstandskraft des Bandes.

Sehr vorteilhaft wird das erfindungsgemäße Band auch bei Kleidungsstücken oder auch therapeutischen Einrichtungen eingesetzt, die sehr lange auf der Hautoberfläche des Trägers verbleiben müssen, beispielsweise Kniekompressen. Dies sind Einrichtungen zur Ruhigstellung von Gelenken, die mit Bändern, die mehrfach um das ruhig zu stellende Glied herum angeordnet werden, auf der Hautoberfläche des Trägers ruhen.

Figur 2 zeigt das in Figur 1 dargestellte elastische Band geschnitten entlang der Linie II-II (und zwar nur in der Schnittebene ohne Hintergrund). Man erkennt in der Figur die leicht auftragenden Haftmittelstreifen 6a, 6b und 6c, die zur Hautoberfläche des Trägers des Bandes zeigen. Das für das hier beschriebene Ausführungsbeispiel verwendete Haftmittel Silikon ist selbstverständlich durch andere zweckmäßige Haftmittel austauschbar.

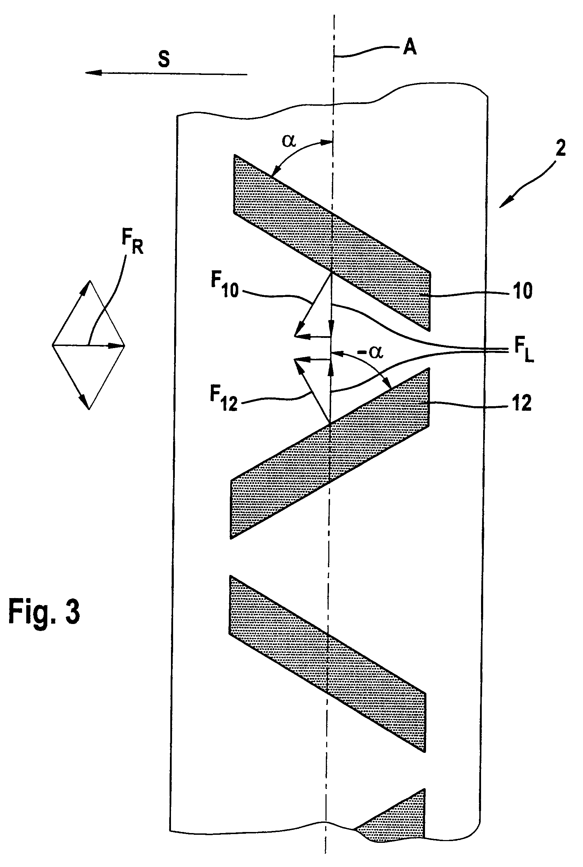

Figur 3 zeigt einen Abschnitt eines elastischen Bands 2 mit zwei benachbarten Haftmittelstreifen 10 und 12, die unter entgegengesetzten Winkeln (α, -α) zur Bandlängsachse A verlaufen. Die Vorteile dieser Ausführungsform der Erfindung ergeben sich wie folgt: Eine Bewegung des Bandes 2 in Richtung des Pfeils S führt zu den Kraftkomponenten F10 und F12, die vektoriell addiert die resultierende Kraft FR ergeben, mit der das Band gehalten wird. Die Kraftkomponenten FL komprimieren dabei das Gewebe des Trägers in Richtung ihrer Kraftlinie, also etwa parallel zur Bandlängsachse A. Damit kommt es zusätzlich zum bekannten Reibschluß vorteilhafterweise zu dem bereits oben diskutierten Formschluß.

## Patentansprüche

1. Elastisches Band mit Antirutschbeschichtung, insbesondere für elastische Strümpfe, dadurch **gekennzeichnet,** daß auf einer Seite des Bandes (2) im wesentlichen quer über die Bandbreite verlaufende, zueinander beabstandete Haftmittelstreifen angeordnet sind.

2. Elastisches Band nach Anspruch 1, dadurch **gekennzeichnet,** daß die Haftmittelstreifen (Streifenabschnitte) (6, 6a, 6b, 6c) im wesentlichen quer zur Bandlängsachse verlaufen und unterbrochen sind.

3. Band nach Anspruch 2, dadurch **gekennzeichnet,** daß die Streifenabschnitte (6, 6a, 6b, 6c) benachbarter Streifen bezüglich der Bandlängsachse zueinander versetzt angeordnet sind.

4. Elastisches Band nach Anspruch 1, dadurch **gekennzeichnet,** daß jeweils zwei benachbarte Haftmittelstreifen (10, 12) unter entgegengesetzten Winkeln (α, -α) zur Bandlängsachse A verlaufen.

5. Kleidungsstück, insbesondere Strumpf, insbesondere therapeutischer Strumpf, **gekennzeichnet** durch wenigstens ein Abschlußband (2) nach einem der vorhergehenden Ansprüche.
